# EUROPEAN PATENT APPLICATION

(11) **EP 2 837 392 A2**
(43) Date of publication of application: **18.02.2015**
(21) Application number: 14182629.7
(22) Date of filing: 15.09.2010
(51) Int. Cl.: A61K 47/48, A61K 9/00, A61K 38/18

(54) **Modified erythropoietin to which water-soluble long-chain molecule is added**

(30) Priority: 15.09.2009 JP 2009213205
(62) Divisional of application: 10817218.0
(71) Applicant: Kaneka Corporation, Kita-ku Osaka 530-8288 (JP)
(72) Inventor: Nobutaka,Tani, Osaka, 530-8288 (JP); Fujii, Toshihide, Hyogo, 676-8688 (JP); Watanabe, Hiroyuki, Hyogo, 676-8688 (JP); Maeda, Hirofumi, Hyogo, 676-8688 (JP)
(74) Representative: Hoffmann Eitle

(57) **Abstract**

An object of the invention is to provide a method of producing a pharmaceutical composition that contains cat or feline erythropoietin (fEPO) as the active ingredient and has, when administered in humans and/or animals, a hematopoietic effect that lasts for not less than seven days. The invention provides a pharmaceutical composition containing, in an amount equal to not less than 50% of the total erythropoietin, erythropoietin to which two or more water-soluble long-chain molecules are added. The invention also provides a pharmaceutical composition containing erythropoietin to which a water-soluble long-chain molecule is added, wherein the water-soluble long-chain molecule has a molecular weight of not less than 30 kDa. The invention further provides a pharmaceutical composition containing erythropoietin to which a water-soluble long-chain molecule is added, wherein the water-soluble long-chain molecule has a branched chain.

SEQUENCE LISTING

<110>  KANEKA CORPORATION

<120>  MODIFIED ERYTHROPOIETIN TO WHICH WATER-SOLUBLE LONG-CHAIN MOLECULE IS
ADDED

<130>  B090354WO01

<140>  PCT/JP2010/065976

<141>  2010-09-15

<150>  JP 2009-213205

<151>  2009-09-15

<160>  1

<170>  PatentIn version 3.1

<210>  1
<211>  192
<212>  PRT
<213>  Felis catus

<400>  1

Met Gly Ser Cys Glu Cys Pro Ala Leu Leu Leu Leu Leu Ser Leu Leu
1               5                   10                  15

Leu Leu Pro Leu Gly Leu Pro Val Leu Gly Ala Pro Pro Arg Leu Ile
            20                  25                  30

Cys Asp Ser Arg Val Leu Glu Arg Tyr Ile Leu Gly Ala Arg Glu Ala
        35                  40                  45

Glu Asn Val Thr Met Gly Cys Ala Glu Gly Cys Ser Phe Ser Glu Asn
    50                  55                  60

Ile Thr Val Pro Asp Thr Lys Val Asn Phe Tyr Thr Trp Lys Arg Met
65                  70                  75                  80

Asp Val Gly Gln Gln Ala Val Glu Val Trp Gln Gly Leu Ala Leu Leu
                85                  90                  95

Ser Glu Ala Ile Leu Arg Gly Gln Ala Leu Leu Ala Asn Ser Ser Gln
            100                 105                 110

Pro Ser Glu Thr Leu Gln Leu His Val Asp Lys Ala Val Ser Ser Leu
        115                 120                 125

Arg Ser Leu Thr Ser Leu Leu Arg Ala Leu Gly Ala Gln Lys Glu Ala
    130                 135                 140

Thr Ser Leu Pro Glu Ala Thr Ser Ala Ala Pro Leu Arg Thr Phe Thr
145                 150                 155                 160

Val Asp Thr Leu Cys Lys Leu Phe Arg Ile Tyr Ser Asn Phe Leu Arg
                165                 170                 175

Gly Lys Leu Thr Leu Tyr Thr Gly Glu Ala Cys Arg Arg Gly Asp Arg
            180                 185                 190

## Description

### TECHNICAL FIELD

The invention relates to a pharmaceutical composition containing erythropoietin to which a water-soluble long-chain molecule is added.

### BACKGROUND ART

To alleviate anemia, erythropoietin (sometimes abbreviated as "EPO"), a hormone which increases hematopoiesis, is produced by genetic recombination techniques and is achieving good results as a pharmaceutical product in the treatment of anemia. It has been demonstrated that adding a polyethylene glycol (sometimes abbreviated as "PEG"), which is a water-soluble long-chain molecule, to human-derived EPO inhibits the metabolism of EPO in the liver, extending the lifetime of EPO in the blood (Patent Document 1). Due to this blood residence time-extending effect, the drug effects last longer, allowing the frequency of administration to be decreased. This advantage has attracted attention to PEG-modified protein drugs as well as to those of EPO as next-generation pharmaceutical products. Indeed, some are already being put to practical use.

When PEG modification is carried out on a protein, the protein has a tendency to be more difficult to metabolize the higher the molecular weight of the PEG added or the greater the number of PEG molecules added. Therefore, it is predicted that a protein to which a large number of high-molecular-weight PEG molecules are added, when administered in vivo, will have a greater longevity in the blood (Non-Patent Document 1). On the other hand, in the case of proteins such as EPO which manifest a physiological activity by binding with a receptor, as the molecular weight of the water-soluble long-chain molecules added becomes higher and the number of such molecules added becomes greater, the physiological activity in vitro decreases.

In a case where the above-mentioned EPO having a human-derived sequence (sometimes abbreviated as "human EPO") was modified with PEG, a PEG-modified EPO in which one molecule of PEG is added to the lysine at position 52 (sometimes abbreviated as "mono-PEGylated form") is reported to have an excellent hematopoietic activity (as indicated by an increase in reticulocytes) prolonging effect when administered in a single dose to rats via the caudal vein (Patent Document 1).

Human EPO preparations in current use are administered by intravenous, subcutaneous, intramuscular and other routes. Additional efforts at developing, for example, oral and nasally absorbed preparations have been made (Patent Document 2). The intravascular administration of human EPO preparations lacks general applicability. Hence, there exists a desire for EPO preparations which are administered by a simpler method, place little strain on the patient, such as less pain following injection, and moreover have a long-lasting drug efficacy.

Anemia symptoms triggered by causes such as chronic renal failure, and chemotherapy and surgery, which are reported to occur in humans, are also observed in animal pets such as dogs and cats, and are often the causes of death in such animals. For example, among cats (within Japan), the incidence of chronic renal failure rose markedly from 0.64% to 3.95% in the ten-year period starting in 1996, climbing from 28th place to 4th place in the ranking of diseases (Non-Patent Document 2). Human EPO preparations are being therapeutically used also to treat anemia in animals. However, because physiologically active proteins such as EPO have species-specific amino acid sequences, when a human-derived EPO preparation is administered to animals other than humans, there is a risk that it will induce the expression of anti-EPO antibodies. As a result, investigations are currently being carried out to develop EPO preparations for the treatment in cats or dogs, each of which has an amino acid sequence that is specific to the particular species.
Patent Document 1: WO 02/32957
Patent Document 2: JP-A S62-89627

Non-Patent Document 1: "Polyethylene glycol-conjugated pharmaceutical proteins," PSTT, Vol'. 1, No. 8, 352-356 (1998).
Non-Patent Document 2: Tama Ju̅i Rinsho̅ Kenkyu̅kai Cho̅sa Ho̅koku (2006 Nendo) [2006 Report on Survey of Tama Veterinary Clinics Association].

### SUMMARY OF THE INVENTION

An object of the invention is to provide a pharmaceutical composition that contains EPO as the active ingredient; has a high safety in vivo although having a high physiological activity; and has a feature that has, when administered in humans and/or animals, a hematopoietic effect that lasts for not less than seven days.

The inventors have conducted careful investigations on the chemical modification of EPO with PEG, as a result of which they have found that, depending on the number of PEG molecules added and the PEG molecular weight or structure, the hematopoietic effect lasts for not less than seven days. Thus the invention has been arrived at.

Accordingly, the invention relates to a pharmaceutical composition comprising an erythropoietin to which two or more water-soluble long-chain molecules are added, wherein an amount of said erythropoietin is not less than 50% of total erythropoietin.

The invention further relates to a pharmaceutical composition containing erythropoietin to which a water-soluble long-chain molecule is added, wherein the water-soluble long-chain molecule has a molecular weight of not less than 30 kDa.

The invention still further relates to a pharmaceutical composition containing erythropoietin to which a water-soluble long-chain molecule is added, wherein the water-soluble long-chain molecule has a branched chain.

The water-soluble long-chain molecule is preferably not less than one compound selected from the group consisting of polyethylene glycol, polyamino acids and polypropylene glycol.

The pharmaceutical composition preferably has a hematopoietic effect in humans and/or animals which lasts for not less than seven days, and the animals are preferably animals of the family Felidae and/or the family Canidae.

Preferably the pharmaceutical composition is a solution or gel, the solution or gel having a pH of not less than 4 but not more than 8.

Preferably, the solution is formulated to have substantially the same osmotic pressure and pH as those of a mammalian bodily fluid so as to give no pain when administered.

The erythropoietin preferably has a human-, cat- or dog-derived amino acid sequence.

The erythropoietin is preferably the following (a) to (c):
(a) a polypeptide having an amino acid sequence set forth in SEQ ID NO:1;
(b) a polypeptide that has an amino acid sequence of not less than 70% sequence identity to the amino acid sequence set forth in SEQ ID NO:1, and has an erythropoietin activity in a cell proliferation assay using BaF/EPOR cells; or
(c) a polypeptide that has one or a plurality of amino acid substitutions, deletions, insertions and/or additions with respect to the amino acid sequence set forth in SEQ ID NO:1, and has an erythropoietin activity in a cell proliferation assay using BaF/EPOR cells.

Preferably, not less than one polyethylene glycol addition site is a lysine-78 residue.

Even when repeatedly administered, the pharmaceutical composition preferably does not trigger the production of an antibody to erythropoietin.

The invention further relates to a drug for treating anemia and a hematopoietic drug, each of which includes the foregoing pharmaceutical composition.

By administering the pharmaceutical composition of the invention in humans or animals, it is possible for the hematopoietic effect to last for not less than seven days. The pharmaceutical composition of the invention, when the preparation is administered, places little strain on the patient, such as less pain following injection, and moreover has a long-lasting drug efficacy.

### BRIEF DESCRIPTION OF THE DRAWINGS

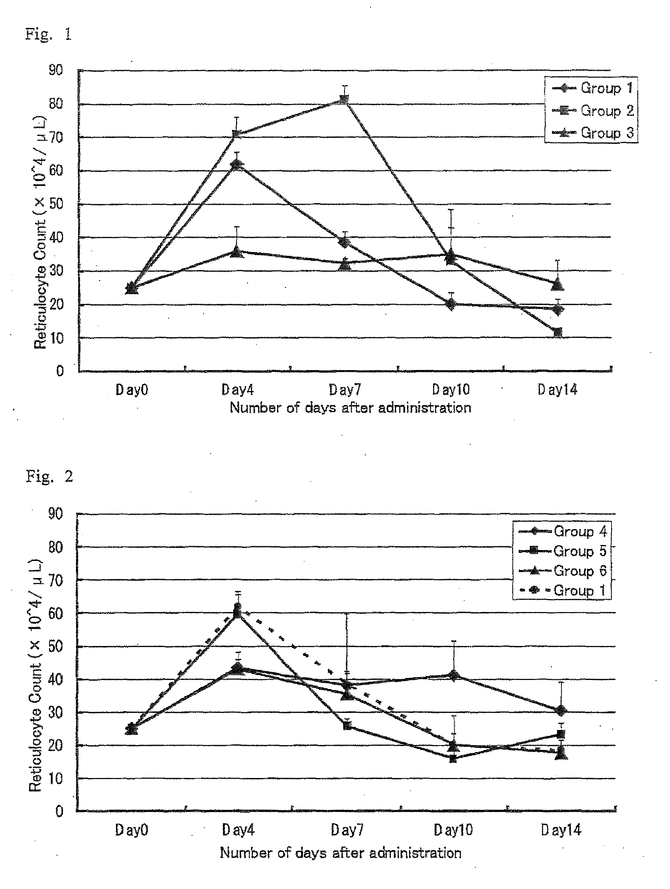

FIG. 1 compares the hematopoietic effects caused when rats were administered PEG-modified cat EPOs in which had been added various numbers of molecules of straight-chain PEG having a molecular weight of 20,000.
FIG. 2 compares the hematopoietic effects caused when rats were administered PEG-modified cat EPOs in which had been added various numbers of molecules of straight-chain PEG having a molecular weight of 5,000 (the control was a mono-PEGylated cat EPO in which had been added one molecule of straight-chain PEG having a molecular weight of 20,000).

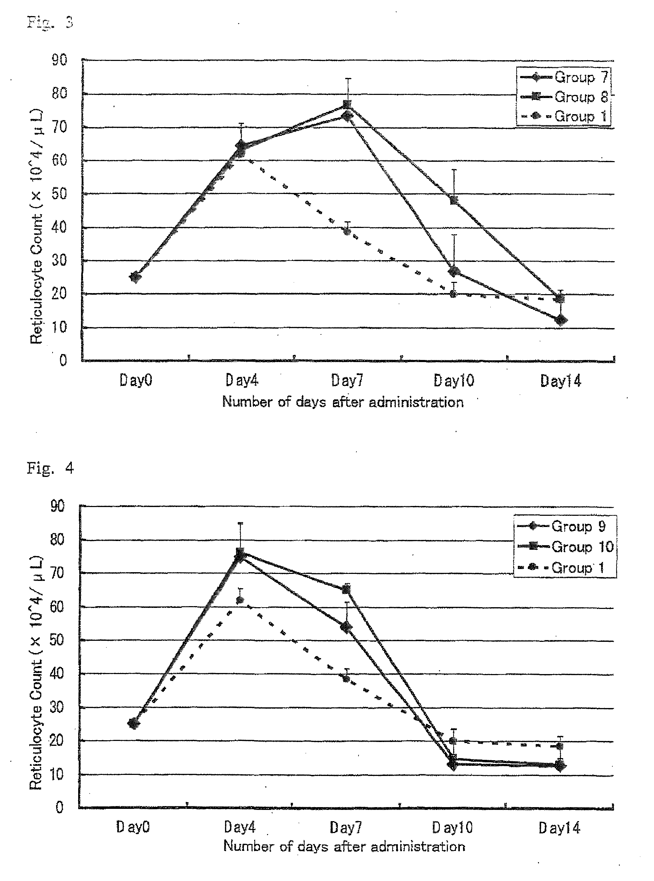

FIG. 3 compares the hematopoietic effects caused when rats were administered PEG-modified cat EPOs in which had been added one or two molecules of straight-chain PEG having a molecular weight of 40,000 (the control was a mono-PEGylated cat EPO in which had been added one molecule of straight-chain PEG having a molecular weight of 20,000).
FIG. 4 compares the hematopoietic effects caused when rats were administered PEG-modified cat EPOs in which had been added one or two molecules of branched PEG having a molecular weight of 20,000 (the control was a mono-PEGylated cat EPO in which had been added one molecule of straight-chain PEG having a molecular weight of 20,000).

### BEST MODE FOR CARRYING OUT THE INVENTION

### 1. The invention relates to three pharmaceutical compositions.

A first aspect of the invention relates to a pharmaceutical composition containing, in an amount equal to not less than 50% of the total erythropoietin, erythropoietin (sometimes abbreviated as "EPO") to which two or more water-soluble long-chain molecules are added.

In the first aspect of the invention, the number of water-soluble long-chain molecules added to EPO is two or more, with two molecules being preferred. EPO to which one water-soluble long-chain molecule is added may additionally be contained. However, from the standpoint of the durability of the hematopoietic effect, EPO to which two or more water-soluble long-chain molecules are added is preferably a main component, more preferably accounts for not less than 50%, and even more preferably accounts for not less than 70%, of the total EPO.

Here, the content of EPO to which two or more water-soluble long-chain molecules are added, based on the total EPO, is calculated by measuring the fluorescent intensity of the bands obtained by fluorescence staining following fractionation by SDS-PAGE.

For EPO to which two or more water-soluble long-chain molecules are added, to be a main component in the total EPO means that EPO to which two or more water-soluble long-chain molecules are added exhibits the most intense band in SDS-PAGE. Here, the phrase "exhibits the most intense band in SDS-PAGE" means that when proteins contained in a sample subjected to electrophoresis by SDS-PAGE are detected by fluorescence staining using, for example, SYPRO Ruby or the like, the intensity of the signal from EPO to which two or more water-soluble long-chain molecules are added is higher than the intensity of other signals.

HPLC may also be used to determine whether EPO to which two or more water-soluble long-chain molecules are added is a main component in the total EPO. For example, a sample passing through a column of, e.g., YMC Pack Diol-200 (available from YMC Co., Ltd.) is detected by absorbance measurement, and the intensity of the signal from EPO to which two or more water-soluble long-chain molecules are added can be determined to be higher than the intensity of other signals.

From the standpoint of the durability of the hematopoietic effect, the molecular weight of the water-soluble long-chain molecule is preferably not less than 10 kDa, and more preferably not less than 20 kDa. At less than 5 kDa, the hematopoietic effect tends not to last long.

A second aspect of the invention relates to a pharmaceutical composition containing EPO to which a water-soluble long-chain molecule is added, wherein the water-soluble long-chain molecule has a molecular weight of not less than 30 kDa.

In the second aspect of the invention, the number of water-soluble long-chain molecules added to EPO is not subject to any particular limitation, and may be one molecule or may be two or more molecules.

From the standpoint of the durability of the hematopoietic effect, the molecular weight of the water-soluble long-chain molecule is preferably not less than 30 kDa, and more preferably not less than 40 kDa. At less than 20 kDa, the hematopoietic effect tends not to last long.

A third aspect of the invention relates to a pharmaceutical composition containing EPO to which a water-soluble long-chain molecule is added, wherein the water-soluble long-chain molecule has a branched chain. By "the water-soluble long-chain molecule has a branched chain," what is meant is that the number of branches is two or more, with the number of branch points being one or more.

In cases where the water-soluble long-chain molecule is a branched chain, the number of water-soluble long-chain molecules added to EPO is not subject to any particular limitation. Even in cases where the number of water-soluble long-chain molecules added is one molecule, the hematopoietic effect caused when the pharmaceutical composition is administered is seen to last for not less than seven days. A method of adding PEG having a branched chain is described in JP-T H9-504299.

The water-soluble long-chain molecule, method and effect of administration, container, and erythropoietin in the foregoing pharmaceutical compositions are described below.

### 2. Water-soluble long-chain molecule

A water-soluble long-chain molecule is added to EPO for the purpose of inhibiting metabolism and prolonging the blood kinetics. The water-soluble long-chain molecule is not subject to any particular limitation, and examples thereof include polyethylene glycol (PEG), polyamino acids and polypropylene glycol. The use of such a molecule may involve preparing a reaction precursor, followed by addition to a protein by way of a synthesis reaction. Genetic recombination techniques may also be used to produce proteins to which these molecules have been linked. Of the above molecules, PEG lacks antigenicity and is nontoxic, and therefore is effective also for lowering the antigenicity of the modified protein and suppressing the expression of anti-EPO antibodies as a side effect.

Methods for covalently linking PEG to a protein generally involve a chemical reaction with a protein, or with an oxidation-activatable functional group on a sugar chain thereof, such as a polyol, lactol, amine, carboxylic acid or carboxylic acid derivative. There are also methods which use a sulfonate ester-activated polymer, such as a sulfonate ester-activated PEG. In the case of addition to EPO as well, addition by such methods is possible.

PEGylation reaction precursors which may be used include long-chain molecules that have been methoxylated at one end. In addition, those which are esterified with a succinimidyl fatty acid at the other unmethoxylated end of PEG have been developed; of these, ones in which the fatty acid part is propionic acid or butyric acid are preferred from the standpoint of reactivity. When succinimidyl propionic acid ester of methoxy PEG (abbreviated as "SPA-PEG") is reacted with human EPO, addition is known to take place selectively at lysine residues. Because a plurality of lysine residues are present on EPO, as the reaction proceeds, the number of PEG molecules added increases, resulting in a mixture of isomers having different numbers of added molecules.

It has been reported that, in the results obtained from the administration of a single dose of human EPO in rats via the caudal vein, a mono-PEGylated form in which a single PEG (20 kDa) was added at lysine 52 had the longest lasting hematopoietic effect; however, optimal isomers or mixture ratio thereof depends on the animal species from which EPO is derived and the route of administration. PEG-modified EPO in which the number of added PEG molecules is one and PEG-modified EPO in which the number of added PEG molecules is two or more may each be administered alone or may be administered in admixture.

The site where PEG is added to the EPO protein will probably depend on the species from which EPO is derived and the mode of reaction. However, even in cases where a plurality of PEG molecules are added, it is preferable for not less than one place of addition to be a lysine residue corresponding to the lysine 52 on human EPO. For example, in cat EPO having the sequence set forth in SEQ ID NO:1, addition at lysine 78 is preferred.

### 3. Method and effect of administration

The EPO-containing pharmaceutical composition of the invention thus has a hematopoietic effect in humans and/or animals which lasts for not less than seven days.

With regard to the hematopoietic effect, the ratio of reticulocytes (erythrocyte precursors) to erythrocytes can be quantitatively determined and used as an indicator of the hematopoietic activity when the inventive pharmaceutical composition has been administered. The reticulocyte count can be measured by a smear test using a dye or stain, or by an automatic blood cell counter.

The subject for administration of the pharmaceutical preparation is not subject to any particular limitation; administration may be carried out in humans and also in animals other than humans. No particular limitation is imposed on the animals other than humans, although animals of the family Felidae and/or the family Canidae are preferred. As will be subsequently described, when a cat-derived EPO is used, in animals of the family Felidae and the family Canidae, the possibility that the EPO will act as an antigen is low, making it possible to avoid the occurrence of side effects.

Methods of administering the pharmaceutical composition include, but are not particularly limited to, intravenous, subcutaneous, oral, intramuscular, percutaneous and nasal administrations.

The PEG-modified EPO in the invention may be used with an agent selected from among pharmacologically acceptable excipients, disintegrants and binders.

Examples of excipients include starch, agar, sucrose, lactose, glucose, dextrin, sorbitol, gum arabic, cornstarch, mannitol, crystalline cellulose, lecithin, calcium phosphate and calcium sulfate. Use may also be made of pharmaceutically acceptable excipients other than these.

Examples of disintegrants include starch, agar, calcium citrate, calcium carbonate, sodium hydrogen carbonate, dextrin, crystalline cellulose, carboxymethylcellulose and tragacanth. Use may also be made of pharmaceutically acceptable disintegrants other than these.

Examples of binders include starch and starch derivatives, cellulose and cellulose derivatives, gum arabic, tragacanth, gelatin, sugars, ethanol and polyvinyl alcohol. Use may also be made of pharmaceutically acceptable binders other than these.

The PEG-modified EPO in the invention may be used with an agent selected from among stabilizers, pH modifiers, osmotic pressure modifiers and surfactants.

Examples of stabilizers include amino acids. Here, amino acids used as stabilizers may be in the form of crystals or may be amorphous. Alternatively, use may be made of those which include impurities, such as plant or animal ingredients containing a high ratio of such amino acids. The crystals used may be in the L form, the D form, or as a mixture of L and D forms.

Examples of pH modifiers include buffer systems selected from the group consisting of acetic acid/acetate, malic acid/malate, citric acid/citrate, tartaric acid/tartrate, lactic acid/lactate, phosphoric acid/phosphate, glycine/glycinate, Tris, glutamic acid/glutamate, and sodium carbonate, and other buffer systems. The lower limit in the pH of a gel or solution containing EPO is preferably 4, more preferably 4.5, and even more preferably 5. The upper limit in the pH is preferably 8, more preferably 7.5, and even more preferably 7.

Examples of osmotic pressure modifiers include, but not limited to, salts, sugars, alcohols and amino acids. In particular, suitable use may be made of sodium chloride, polyhydric alcohols, monohydric alcohols, monosaccharides, disaccharides, oligosaccharides and amino acids, as well as derivatives thereof.

Examples of polyhydric alcohols that may be used include trihydric alcohols such as glycerol; pentahydric alcohols such as arabitol, xylitol and adonitol; and hexahydric alcohols such as mannitol, sorbitol and dulcitol. Of these, hexahydric alcohols are preferred, and mannitol is especially suitably used.

Examples of the monohydric alcohols include methanol, ethanol and isopropyl alcohol. Of these, ethanol is preferred.

Examples of the monosaccharides that may be used include five-carbon sugars (pentoses) such as arabinose, xylose, ribose and 2-deoxyribose; and six-carbon sugars (hexoses) such as glucose, fructose, galactose, mannose, sorbose, rhamnose and fucose. Of these, six-carbon sugars are preferred.

Examples of the oligosaccharides that may be used include trisaccharides such as maltotriose and raffinose, and tetrasaccharides such as stachyose. Of these, trisaccharides are preferred.

Examples of derivatives of these monosaccharides, disaccharides and oligosaccharides that may be used include glucosamine, galactosamine, glucoronic acid and galacturonic acid.

In addition, a surfactant may be contained with the PEG-modified EPO in the invention. Exemplary surfactants include anionic surfactants, nonionic surfactants, amphoteric surfactants and cationic surfactants, although the possibilities are not limited to these. Exemplary anionic surfactants include anionic surfactants based on fatty acids, anionic surfactants based on linear alkylbenzenes, anionic surfactants based on higher alcohols, anionic surfactants based on α-olefins and anionic surfactants based on normal paraffins, but are not limited to these. Exemplary nonionic surfactants include nonionic surfactants based on fatty acids, nonionic surfactants based on higher alcohols and nonionic surfactants based on alkylphenols, but are not limited to these. Illustrative, non-limiting, examples of nonionic surfactants include polysorbate and/or polyoxyethylene glycol sorbitan alkyl esters. Exemplary amphoteric surfactants include amphoteric surfactants based on amino acids, betaines, or amine oxides, but are not limited to these. Exemplary cationic surfactants include cationic surfactants based on quaternary ammonium salts, but are not limited to these.

These above excipients, disintegrants, binders, stabilizers, pH modifiers, osmotic pressure modifiers and surfactants may be freely combined. In addition, pharmaceutically acceptable additives other than excipients, disintegrants, binders, stabilizers, pH modifiers, osmotic pressure modifiers and surfactants may be added. Examples thereof include lubricants, coating agents, colorants, dispersing agents, absorption promoters, solubilizing agents, health food materials, dietary supplement materials, vitamins, fragrances, sweeteners, antiseptics, preservatives and antioxidants.

To obtain the pharmaceutical preparation, the ingredients may be processed as a solution, gel or powder, and subsequently rendered into a solution-type preparation, a lyophilized preparation, a prefilled syringe, slow-release preparation for subcutaneous implantation, a micelle preparation, a gel preparation, or a liposome preparation for example.

When an EPO-containing pharmaceutical composition is used as a solution, it is important for the solution to be formulated to have substantially the same osmotic pressure and pH as those of a mammalian bodily fluid (osmotic pressure: 280 mOsm/Kg H₂O; pH: 7.4) so as to give no pain at the time of subcutaneous administration. When the EPO-containing pharmaceutical composition is used as a solution, those having an osmotic pressure of not more than 400 mOsm/Kg H₂O can be suitably used in the invention. Also, those having an osmotic pressure of not less than 200 mOsm/Kg H₂O can be suitably used.

### 4. Container

Because EPO is effective in vivo, even when administered in a small dose, losses from adsorption of the preparation to the container wall exert a large influence on the effective activity when administered in vivo. The container for this preparation is thus preferably a resin product to which little protein adsorbs. Accordingly, it is desirable to select a container material such that EPO adsorption to the container is not more than 1% of the total amount of EPO in the container. It is advantageous to select a material such that such adsorption is preferably not more than 0.7%, and more preferably not more than 0.5%.

No particular limitation is imposed on the resin materials which are known to have a low protein adsorption. Examples thereof include container materials for medical use such as polyethylene (PE), polypropylene (PP), polyethylene terephthalate (PET), polycarbonate and polyethyl methacrylate. Preferred examples of the resin materials that are known to have a low protein adsorption include resins which are polymers obtained by ring-opening polymerizing a cycloolefin such as norbornene, tetracyclododecene or a derivative thereof, and hydrogenates of these polymers; and copolymers in which a cyclopentyl residue or substituted cyclopentyl residue has been inserted in the molecular chain by polymerizing a cycloolefin such as norbornene, tetracyclododecene or a derivative thereof with ethylene or propylene. Cycloolefin copolymers (COC), which are copolymers obtained from tetracyclododecene and an olefin such as ethylene as the starting materials, are more preferable in that they have a low adsorption. In addition, cycloolefin polymers (COP), which are polymers obtained by the ring-opening polymerization of norbornene and then hydrogenation, are similarly preferred (see JP-A H5-300939 or JP-A H5-317411).

### 5. Amino acid sequence of EPO

The amino acid sequence of EPO may be a sequence derived from any organism. As well as humans, the cDNA cloning of EPO has been carried out in, for example, mice, rats, dogs and cats (Wen et al.: Blood 82, 1507 (1993)), and the amino acid sequences coding for these EPOs have been elucidated. In the invention, it is possible to use not only human EPO, but also EPOs having amino acid sequences specific to species other than humans.

The amino acid sequence of EPO in the invention is preferably a human-derived amino acid sequence or an amino acid sequence derived from an animal of the order Carnivora. Within the order Carnivora, the family Felidae and the family Canidae are preferred, with the family Felidae being more preferred.

Cat EPO is known to have 83.4% sequence identity to human EPO. Also, cat EPO expressed in CHO cells has a hematopoietic activity in cats (Am. J. Vet. Res. 64, 1465-71 (2003)).

Causes of anemia are known to include massive hemorrhaging, vitamin insufficiency, autoimmune diseases, malignant tumors, chronic inflammation, chronic renal failure, hemolysis and hematopoietic abnormality. Anemia from these causes occurs not only in humans, but also in pets such as dogs and cats, in livestock such as cattle, horses, sheep, goats and pigs, and even in animals such as lions, tigers, kangaroos, elephants, giraffes, zebras, koalas and pandas which are raised and publicly exhibited for ecological study in zoos and the like. Such cases of anemia can be treated with the EPO used in the invention. Cat EPO may be used to treat anemia in animals of the family Felidae, such as cats, lions and tigers. Moreover, cat EPO has a high sequence identity to dog EPO, and is also effective in dogs and other animals of the family Canidae, which belong taxonomically to the order Carnivora.

Unlike humans, because there is no blood bank system for transfusion in the aforementioned animals, it is difficult to adopt blood infusion measures for blood loss due to an accident or during surgery for example. However, the EPO preparation of the invention which has a long-lasting hematopoietic effect can be used in such animals, either in place of transfusion or at the time of drip infusion. If an animal feels pain during administration, this may hinder administration and, in the case of large animals, may even pose a danger to the veterinarian. However, pain can be minimized by adjusting the osmotic pressure and pH to values close to those in the bodily fluid of the subject animal.

The amino acid sequence of EPO derived from animals of the family Felidae is preferably an amino acid sequence set forth in SEQ ID NO:1.

A polypeptide having an amino acid sequence of not less than 70% sequence identity to the amino acid sequence making up the EPO as shown in SEQ ID NO:1 is also included in the inventive EPO.

The sequence identity of the EPO polypeptide to the amino acid sequence set forth in SEQ ID NO:1 is preferably not less than 70%, more preferably not less than 80%, even more preferably not less than 85%, still more preferably not less than 90%, and most preferably not less than 95%.

Alternatively, the EPO may be a polypeptide in which one or a plurality of amino acids have been substituted, deleted, inserted and/or added, provided the hematopoietic activity is not lost. The number of such changes introduced is preferably not more than 50, more preferably not more than 40, even more preferably not more than 30, still more preferably not more than 20, and most preferably not more than 10.

With regard to the method of producing EPO, in addition to recombinant-produced EPO which is produced with commonly available animal cells (e.g., CHO cells), prokaryotic organisms or yeasts as the host, use can also be made of EPO such as those collected from a natural source and those produced in recombinant animals, although the possibilities are not limited to these. It is also possible to utilize transgenic birds as the recombinant animals which produce EPO. Either the purified product or product in an unpurified state is acceptable, although the purified product is preferred from the standpoint of quality control.

Purification of EPO may be carried out using common protein recovery techniques such as salting out, adsorption column chromatography, ion exchange column chromatography, gel filtration column chromatography and antibody column methods, either alone or in combination, although the possibilities are not limited only to these. Examples of adsorption column chromatography include blue sepharose chromatography and heparin chromatography. Examples of ion exchange column chromatography include cation exchange chromatography and anion exchange chromatography.

### EXAMPLES

The invention is described in more detail by way of the following examples, although the invention is not limited to these examples. In cases where trade names are mentioned, unless noted otherwise, the instructions in the accompanying user's manual were followed.

The synthesis of cat-derived erythropoietin was carried out by the process described in JP-A 2007-89578. The steps in that process are recited once again below.

### Preparation Example 1: Microinjection of Retroviral Vector in Chicken Embryo and Artificial Hatching

A retroviral vector for expressing cat-derived erythropoietin was microinjected into a chicken embryo, thereby producing a transgenic chicken which expresses cat-derived erythropoietin. Microinjection and artificial hatching were carried out under aseptic conditions. Fertilized chicken eggs (available from Shixoyama Shukeijo) were disinfected on the outside with a disinfectant (available from Showa Furanki) and ethanol. An incubator (model P-008(B), available from Showa Furanki) was adjusted to an environment of 38°C and 50-60% humidity and, with the incubation starting time (0 hours) being the time at which the power was turned on, incubation was carried out while rotating the eggs 90° every 15 minutes thereafter.

When about 55 hours had elapsed from the start of incubation, the eggs were removed from the incubator, the pointed ends were cut away in the form of a circle of 3.5 cm diameter with a mini-router (available from Proxxon) fitted with a diamond edge (edge diameter, 20 mm; shaft diameter, 2.35 mm). The contents of the fertilized eggs were transferred to egg shells prepared by cutting away the pointed ends of double-yolked chicken eggs (available from Shiroyama Shukeijo) to a diameter of 4.5 cm and discarding the contents, and the embryos were moved upward with a syringe plunger. A viral solution was poured into Femtotips II (available from Eppendorf) under a stereomicroscope system (SZX12, available from Olympus Corporation) and about 2 µL of the solution containing a retroviral vector for expressing cat-derived erythropoietin as mentioned in JP-A 2007-89578 was microinjected using a FemtoJet (available from Eppendorf).

Using egg white as the glue, each of these openings was sealed with a piece of Saran wrap (available from Asahi Kasei Corporation) cut to a size of about 8×8 cm², following which the eggs were returned to the incubator and incubation was continued. Egg rotation in the incubator was changed to 30° every 30 minutes. On day 20 from the start of incubation, about 20 holes were made in the Saran wrap with a 20G syringe needle and incubation was then carried out while supplying 60 cc/min of oxygen to the incubator. Once the chicks in the eggs began pipping, the shells were broken, allowing the chicks to hatch. The chicks that emerged were raised and allowed to grow. SX Safety and Neo-Safety 17 for young chicks (available from Toyohashi Feed Mills Co., Ltd.) were used as the feed. The expression of cat-derived erythropoietin in the blood and eggs of transgenic chickens was confirmed by the subsequently described cell proliferation assay using

BaF/EPOR.

### Preparation Example 2: Purification of Cat-Derived Erythropoietin from Egg White

The eggs of individual chickens for which cat-derived erythropoietin activity had been confirmed in the egg white were collected. Using the subsequently described columns, cat-derived erythropoietin was collected from the egg white and purified.

The samples to be applied to a column were all syringe filtered, just prior to use, with a Millex-HV having a pore size of 0.45 µm (available from Nihon Millipore). When filtration was difficult, pre-filtration was carried out with a Puradisc 25 having a pore size of 2 µm (available from Whatman Ltd.), following which filtration with the Millex was carried out.

Measurement of the cat-derived erythropoietin content in the process of purification was carried out with a Biacore 3000 system (available from GE Healthcare Japan, BIACORE). Anti-human erythropoietin monoclonal antibodies (available from R&D Systems) were subjected to NHS immobilization on research-grade CM5 Sensor Chips (available from GE Healthcare Japan, BIACORE) using an amine coupling kit (available from GE Healthcare Japan, BIACORE) to form chips for measurement, and the concentration was then measured with Epogin as the standard substance and using the assay program on the system.

In order to apply the egg white to the column, pretreatment to lower the viscosity was carried out. The egg which had been refrigerated was returned to room temperature, and cracked open, and the egg yellow and egg white were separated using the egg shell or the like, following which only the egg white was collected and weighed. The egg white was mixed with a stirrer, thereby loosening up the viscous egg white, after which a five-fold quantity of ultrapure water was added and further mixing was carried out. The pH of the egg white solution at this time was about 9.0 to 9.3. A suitable amount of 1N HCl was added to adjust the pH to 5.0 and stirring was carried out for not less than 15 minutes, following which 30 minutes of centrifugal separation was carried out at 9,500 G and 4°C. Next, 1M NaOH was added to the supernatant, thereby adjusting the pH to 7.0, and then 1M Tris buffer (pH 7.0) was added to a final concentration of 50 mM. The maximum recovery of cat-derived erythropoietin in this step was 95%.

Next, blue sepharose chromatography was carried out. An amount of 500 mL of the pre-treated egg white solution (equivalent to 2 to 3 egg whites) was applied to a 50 mL Blue Sepharose 6 Fast Flow column (available from GE Healthcare Japan, Amersham) that had been equilibrated with 50 mM Tris (pH 7.0). The column was thoroughly washed with 50 mM Tris (pH 7.0), and then eluted with 200 mL of 1M NaCl and 50 mM Tris (pH 7.0). The eluted fractions were dialyzed overnight by a standard method with 20 mM MES (pH 6.2) in a low-temperature chamber at 4°C, thereby carrying out buffer exchange. The maximum recovery of cat-derived erythropoietin in this step was 98%.

Next, heparin chromatography was carried out. The blue sepharose-eluted fraction (following dialysis) was applied in two divided portions to a HiPrep 16/10 Heparin FF column (available from GE Healthcare Japan, Amersham) that had been equilibrated with 20 mM MES (pH 6.2) and the column was thoroughly washed each time with 20 mM MES (pH 6.2), following which gradient elution up to an NaCl concentration of 80 mM was carried out. The column was regenerated each time with 1 M NaCl and 0.1 M NaOH. Those fractions in which the presence of cat-derived erythropoietin was confirmed by the Biacore system were collected. The maximum recovery of cat-derived erythropoietin in this step was 80%.

Next, buffer exchange was carried out with a desalting column. The heparin sepharose-eluted fractions was concentrated to a total volume of about 30-40 mL with a Vivaspin 20 (available from Sartorius Mechatronics Japan) having a molecular weight cutoff of 5,000, applied 10 mL at a time to a HiPrep 26/10 Desalting column (available from GE Healthcare Japan, Amersham) equilibrated with 25 mM Tris (pH 7.0), and eluted with the same buffer and the protein-containing fraction was recovered. The pH was adjusted to 9.0 with 1 M NaOH, in addition to which the electric conductivity was adjusted to 3.0 to 3.2 mS/cm with 1 M NaCl. The maximum cat-derived erythropoietin recovery in this step was 95%.

Next, anionic exchange column chromatography was carried out. The sample following buffer exchange was applied in two divided portions to a 5 mL HiTrap DEAE FF column (available from GE Healthcare Japan, Amersham) that had been equilibrated with 25 mM Tris (pH 9.0) and at an electrical conductivity of 3.0 to 3.2 mS/cm, and a fraction of each portion that passed through the column without adsorption was collected. The column was regenerated each time with 1 M NaCl. The fractions were concentrated to a total volume of about 2-3 mL with a Vivaspin 20 having a molecular weight cutoff of 5,000. The maximum cat-derived erythropoietin recovery in this step was 92%.

Next, gel filtration chromatography was carried out. The concentrated sample was applied to a Superdex 200 10/300 GL column (available from GE Healthcare Japan, Amersham) equilibrated with a 50 mM borate buffer (pH 9.0) or another suitable buffer, and was eluted with the same buffer. Those fractions in which the presence of cat-derived erythropoietin was confirmed by the Biacore system were collected, and then concentrated to a total volume of about 1-2 mL with a Vivaspin 6 having a molecular weight cutoff of 5,000. The maximum cat-derived erythropoietin recovery in this step was 93%.

SDS-PAGE was carried out on the fractions recovered in the purification steps. The samples were electrophoresced under denaturation conditions using a 12.5% e-PAGEL, and detected with a Bio-Safe Coomassie Stain (available from Bio-Rad Laboratories).

A Baf/EPOR cell proliferation assay was carried out by the subsequently described method on the cat-derived erythropoietin purified from egg white. As a result, the specific activity was 160,000 to 290,000 IU/mg.

PEG addition to the cat-derived erythropoietin was carried as follows.

### Example 1: Reaction for Synthesizing Cat-Derived Erythropoietin to Which PEG is added (PEGylated Cat EPO)

Using the purified cat EPO solution (20 mM phosphate buffer, pH = 7.0), the following PEG reagents were added.
Straight-chain PEG having a molecular weight of 5,000 (available from NOF Corporation; ME-050HS)
Straight-chain PEG having a molecular weight of 20,000 (available from NOF Corporation; ME-200HS)
Straight-chain PEG having a molecular weight of 40,000 (available from NOF Corporation; ME-400HS)
Branched PEG having a molecular weight of 20,000 (available from NOF Corporation; GL2-200GS2; number of branch points: 1)

The cat EPO and PEG reagent were mixed together in a molar ratio of 1:5, and then reacted while being intimately mixed at 4°C. Because with PEG addition, first a mono-PEGylated form, next a di-PEGylated form, then an oligo-PEGylated form are generated, the generation of the respective PEGylated forms was monitored by HPLC (available from Shimadzu Corporation), and the reaction was stopped at a stage where sufficient amounts had been obtained. A YMC Pack Diol-200 (normal phase system; available from YMC Co., Ltd.) was used as the column.

When sufficient amounts of PEGylated forms had been generated, a 1/10th amount of a 100 mM glycine solution was added as a reaction stopper to stop the reaction while carrying out intimate mixing at 4°C for 1 hour, following which the reaction mixture was subjected to dialysis (against a 50 mM acetic acid buffer, pH = 4.5).

### Example 2: PEGylated Cat EPO Separation and Purification by PEG Reaction Mixture Purification

In order to separate and collect the mono-PEGylated di-PEGylated and oligo-PEGylated forms generated by PEG reactions and the unreacted PEG and unreacted EPO, separation and purification were carried out with a cation-exchange column.

The reaction mixture was subjected to separation and purification (bonding: 50 mM acetic acid buffer (pH = 4.5); elution: 1 M NaCl gradient) using a cation-exchange column (MacroCap SP, available from GE Healthcare Japan). The peaks of those eluted and isolated with salt concentrations in the order of oligo-PEGylated, di-PEGylated and mono-PEGylated forms were separately collected, and each was dialyzed (against 20 mM phosphate buffer (pH = 7.5), 150 mM NaCl). Thereto was added 0.05% (v/v) of Polysorbate 80 (available from Wako Pure Chemical Industries, Ltd.) as a dispersant, thereby giving a sample for administration.

### Evaluation 1: Measurement of Cat EPO and PEGylated Cat EPO Activities

The in vitro activities of the various PEGylated cat EPOs synthesized in Example 2 were measured.

Measurement of the cat EPO activities was carried out by a cell proliferation assay (JP-A H10-94393) using BaF/EPOR cells (The Chemo-Sero-Therapeutic Research Institute (Kaketsuken)), which is an EPO-dependent cell line. In the cell proliferation assay, a working curve of proliferation was created using Epogin (available from Chugai Pharmaceutical Co., Ltd.) as the standard erythropoietin, and the EPO activities of unknown samples were measured based on the working curve. An RPMI 1640 liquid medium (available from Nissui Pharmaceutical Co., Ltd.) containing 5% fetal bovine serum (FBS) and 50 units/mL penicillin and streptomycin was used as the medium for BaF/EPOR cells. During the normal cultivation of BaF/EPOR cells, Epogin was added to a final concentration of 1 U/mL. Cells in the logarithmic growth phase were used in the cell proliferation assay.

In order to carry out a cell proliferation assay with BaF/EPOR cells, first the Epogin within the medium was removed. The cultured BaF/EPOR cells were centrifugally separated for a period of 5 minutes at 1,000 rpm. The supernatant was removed, and 10 mL of Epogin-free medium was added to the precipitate, which was suspended therein. The same operation was carried out three times, thereby removing the Epogin within the medium. The cells were counted, and diluted with Epogin-free medium to a concentration of 55,555 cells/mL. The diluted suspension was sown in an amount of 90 µL per well onto a 96-well microtiter plate. Thereto was added Epogin diluted to concentrations of 25, 16, 10, 6.4, 4.0, 2.5, 1.6 and 1.0 U/mL in medium in amounts of 10 µL per well, and the cells were uniformly suspended therein (the final EPO concentrations were 2.5, 1.6, 1.0, 0.64, 0.4, 0.25, 0.16 and 0.1 U/mL, respectively).

The samples used in the assay were serially diluted about 2- to 4-fold per step with medium to fall within the measurement range of the working curve, and then 10 µL portions of each sample dilution were added to the sown cells, following which the cells were uniformly suspended. Triplicate measurements were performed for each standard sample or unknown sample. Culturing was carried out for two days and 10 µL of a Cell Counting Kit-8 solution (available from Dojindo Laboratories) was then added to each well. A color reaction was carried out for 1 to 4 hours, following which the reaction was stopped by adding 10 µL of 0.1 mol/L hydrochloric acid, and the 450 nm absorbance was measured using a microplate reader. An approximation formula was derived from the measurement results for the standard samples by a logarithmic approximation. The activity of each sample was calculated based on the approximation formula obtained.

The measurement results are shown in Table 1.

**[Table 1]**

| In vitro specific activity (I.U./mg) | | | | |
|---|---|---|---|---|
| PEG Reagent | | Type of PEGylated cat EPO formed | | |
| Structure | Molecular weight | mono | Di | oligo |
| Straight chain | 5K | 53,866 | 8,443 | 814 |
| Straight chain | 20K | 24,320 | 1,670 | ND ( 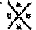 ) |
| Straight chain | 40K | 7,268 | 289 | ND ( 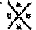 ) |
| Two branches | 20K | 6,392 | 546 | ND ( 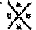 ) |
| Epogin | | 200,000( 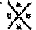 ) | | |

| | | | | |
|---|---|---|---|---|
| 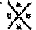 ND=not detected 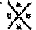 Data from interview form | | | | |

### Evaluation 2: Subcutaneous Administration Test in Rats

The samples prepared in Example 2 were subcutaneously administered in rats.

The sample solution was subcutaneously administered in a single dose of 303 µg/kg in male rats (available from Charles River Japan, SPF, 7 week old). The experimental groups and the samples for administration are shown in Table 2.

**[Table 2]**

| | Sample for administration | | Dose (*µ*g/kg) | n (number) | Administration route |
|---|---|---|---|---|---|
| | Molecular weight and structure of added PEG molecule(s) | Number of added PEG molecules | | | |
| Group 1 | 20kDa, Straight-chain | Mono | 303 | 3 | Subcutaneous |
| Group 2 | | Di | 303 | 3 | Subcutaneous |
| Group 3 | | Oligo | 303 | 3 | Subcutaneous |
| Group 4 | 5kDa, Straight-chain | Mono | 303 | 3 | Subcutaneous |
| Group 5 | | Di | 303 | 3 | Subcutaneous |
| Group 6 | | Oligo | 303 | 3 | Subcutaneous |
| Group 7 _ | 40kDa, Straight-chain | Mono | 303 | 3 | Subcutaneous |
| Group 8 | | Di | 303 | 3 | Subcutaneous |
| Group 9 | 20kDa, Branched | Mono | 303 | 3 | Subcutaneous |
| Group 10 | | Di | 303 | 3 | Subcutaneous |

| | | | | | |
|---|---|---|---|---|---|
| 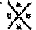 All PEGs used are available from NOF Corporation. | | | | | |

### Evaluation 3: Measurement of Rat Reticulocyte Count

Letting the time just prior to subcutaneous administration be Day 0, blood was sampled from the cervical vein serially on Days 4, 7, 10 and 14 following subcutaneous administration. Using the sampled whole blood, the reticulocyte count in peripheral blood was measured (Methylene Blue staining method: Laboratory-Network-Systems Inc.). Changes in the reticulocyte count are shown in Figs. 1 to 4.

### Evaluation 4: Method of Measuring Content of Erythropoietin to which Water-Soluble Long-Chain Molecule(s) are Added

A solution of erythropoietin to which water-soluble long-chain molecule(s) are added is dialyzed against a 20 mM phosphate buffer (pH = 7.5). Using a spectrophotometer (Gene Quant pro; available from GE Healthcare Japan, Amersham; code 80-2114-98) and UV cells, and employing a 20 mM phosphate buffer (pH = 7.5) as the blank, the solution is adjusted with the phosphate buffer so that the absorbance at a wavelength of 280 nm becomes 0.1.

To the solution is added an equivalent amount of Laemmli sample buffer (available from Bio-Rad Laboratories, code 161-0737; to which DTT has been added to a final concentration of 350 mM), and heat denaturation at 95°C is carried out for 5 minutes, following which the resulting solution is rapidly cooled on ice to give a sample, of which 2 µL is electrophoresced. Electrophoresis is carried out for 80 minutes using an e-PAGEL gel (E-R12.5L, available from Atto Corporation), using a pageRUN electrophoresis system (Model AE-6531, available from Atto Corporation), and after setting the current to 20 mA per slab gel. When electrophoresis has ended, the gel is peeled from the gel plate and immersed for 30 minutes in ultrapure water (Milli-Q, available from Nihon Millipore, MILLIPORE ZMQS7V0T1). The ultrapure water is removed and the gel is fixed by 30 minutes of immersion in a fixing solution. The fixing solution is removed, and a staining solution is added, then the gel is immersed therein for not less than 3 hours but less than 6 hours. The staining solution is removed, and the gel is then immersed in a fixing solution and washed for 60 minutes while being gently shaken, after which the fixing solution used for washing is removed. A fresh fixing solution is then added, and 60 minutes of washing is carried out under gentle shaking.

After two washes, an image of the gel is captured while carrying out ultraviolet irradiation with a ChemiDoc XRS system (Bio-Rad Laboratories). Using the software Quantity One, ver. 4.6 (Bio-Rad Laboratories), the fluorescent intensity of the bands in the image is measured. After defining lanes using the Frame Lanes command, the content is computed from the ratio of the area intensity displayed using the Lane Background command. In cases where a plurality of bands are found to exist, the ratio of the fluorescent intensity of the band having the target molecular weight to the sum of the fluorescent intensities of all the bands is defined as the content ratio.

The 20 mM phosphate buffer (pH = 7.5), fixing solution and staining solution used are shown below.

### (i) 20 mM Phosphate Buffer (pH = 7.5)

Obtained by dissolving the following reagents in 1 L of ultrapure water:
0.59 g of sodium dihydrogen phosphate dihydrate (available from Wako Pure Chemical Industries, Ltd.; 192-0825),
5.3 g of disodium hydrogen phosphate dodecahydrate (available from Wako Pure Chemical Industries, Ltd.; 196-02835), and
8.76 g of sodium chloride (available from Nacalai Tesque, Inc.; 31320-05).

### (ii) Fixing Solution

An aqueous solution prepared at the following concentrations using ultrapure water:
10% methanol (available from Nacalai Tesque, Inc.; 21915-93), and
7% acetic acid (available from Nacalai Tesque, Inc.; 00212-85).

### (iii) Staining Solution

SYPRO Ruby Protein Gel Stain (Lonza; Cat. No. 50564)

In the following further embodiments of the invention are described:
1. A pharmaceutical composition comprising an erythropoietin to which two or more water-soluble long-chain molecules are added, wherein an amount of said erythropoietin is not less than 50% of total erythropoietin.
2. A pharmaceutical composition comprising erythropoietin to which a water-soluble long-chain molecule is added, wherein the water-soluble long-chain molecule has a molecular weight of not less than 30 kDa.
3. A pharmaceutical composition comprising erythropoietin to which a water-soluble long-chain molecule is added, wherein the water-soluble long-chain molecule has a branched chain.
4. The pharmaceutical composition according to any one of items 1 to 3, wherein the water-soluble long-chain molecule is not less than one compound selected from the group consisting of polyethylene glycol, polyamino acids and polypropylene glycol.
5. The pharmaceutical composition according to any one of items 1 to 4, which has a hematopoietic effect in humans and/or animals which lasts for not less than seven days.
6. The pharmaceutical composition according to item 5, wherein the animals are animals of the family Felidae and/or the family Canidae.
7. The pharmaceutical composition according to any one of items 1 to 6, which is a solution or gel, the solution or gel having a pH of not less than 4 but not more than 8.
8. The pharmaceutical composition according to item 7, wherein the solution is formulated to have substantially the same osmotic pressure and pH as those of a mammalian bodily fluid so as to give no pain when administered.
9. The pharmaceutical composition according to any one of items 1 to 8, wherein the erythropoietin has a human-, cat- or dog-derived amino acid sequence.
10. The pharmaceutical composition according to any one of items 1 to 9, wherein the erythropoietin is the following (a) to (c):
   (a) a polypeptide having an amino acid sequence set forth in SEQ ID NO:1;
   (b) a polypeptide that has an amino acid sequence of not less than 70% sequence identity to the amino acid sequence set forth in SEQ ID NO:1, and has an erythropoietin activity in a cell proliferation assay using BaF/EPOR cells; or
   (c) a polypeptide that has one or a plurality of amino acid substitutions, deletions, insertions and/or additions with respect to the amino acid sequence set forth in SEQ ID NO:1, and has an erythropoietin activity in a cell proliferation assay using BaF/EPOR cells.
11. The pharmaceutical composition according to item 10, wherein not less than one polyethylene glycol addition site is a lysine-78 residue.
12. The pharmaceutical composition according to any one of items 1 to 11, wherein even when repeatedly administered, the pharmaceutical composition does not trigger the production of an antibody to erythropoietin.
13. A drug for creating anemia, comprising the pharmaceutical composition according to any one of items 1 to 12.
14. A hematopoietic drug, comprising the pharmaceutical composition according to any one of items 1 to 12.

## Claims

1. A method of producing a pharmaceutical composition comprising a modified erythropoietin, wherein the method comprises the following steps (A) and (B):
(A) producing erythropoietin in a host that has a gene coding for a polypeptide that has an amino acid sequence as set forth in SEQ ID NO:1,
wherein the host is selected from animal cells, prokaryotic organisms, yeasts and recombinant animals, and
(B) adding two or more water-soluble long-chain molecules to not less than 50% of the total erythropoietin.

2. The method according to claim 1, wherein the host for producing erythropoietin is selected from CHO cells and transgenic birds.

3. The method according to claim 1 or 2,
wherein the water-soluble long-chain molecule is not less than one compound selected from the group consisting of polyethylene glycol, polyamino acids and polypropylene glycol.

4. The method according to any one of claims 1 to 3,
wherein the pharmaceutical composition has a hematopoietic effect in humans and/or animals which lasts for not less than seven days.

5. The method according to claim 4,
wherein the animals are animals of the family Felidae and/or the family Canidae.

6. The method according to any one of claims 1 to 5,
wherein the pharmaceutical composition is a solution or gel, the solution or gel having a pH of not less than 4 but not more than 8.

7. The method according to claim 6,
wherein the solution is formulated to have substantially the same osmotic pressure and pH as those of a mammalian bodily fluid so as to give no pain when administered.

8. The method according to any one of claims 1 to 7,
wherein not less than one polyethylene glycol addition site is a lysine-78 residue.

9. The method according to any one of claims 1 to 8,
wherein even when repeatedly administered, the pharmaceutical composition does not trigger the production of an antibody to erythropoietin.

10. The method according to any one of claims 1 to 9,
wherein the water-soluble long-chain molecule is straight-chain polyethylene glycol of 5-40 kDa.

11. A pharmaceutical composition,
wherein the composition comprises a modified erythropoietin to which two or more water-soluble long-chain molecules have been added,
wherein an amount of said modified erythropoietin is not less than 50% of total erythropoietin, and
wherein the erythropoietin is a polypeptide that has 20 to 30 amino acid deletions with respect to the amino acid sequence set forth in SEQ ID NO:1, and has an erythropoietin activity in a cell proliferation assay using BaF/EPOR cells.

12. A pharmaceutical composition,
wherein the composition comprises a modified erythropoietin to which two or more water-soluble long-chain molecules have been added,
wherein an amount of said modified erythropoietin is not less than 50% of total erythropoietin,
wherein the erythropoietin is producible in a host that has a gene coding for a polypeptide that has an amino acid sequence as set forth in SEQ ID NO:1, and
wherein the host is selected from animal cells, prokaryotic organisms, yeasts and recombinant animals.

13. The pharmaceutical composition according to claim 11 or 12, wherein the composition is obtainable by any one of the methods of claims 1 to 10.

14. The pharmaceutical composition according to any one of claims 11 to 13 for use as a pharmaceutical.

15. A drug for use in a method of treating anemia,
wherein the drug comprises the pharmaceutical composition according to any one of claims 11 to 13.

16. A drug for use as a hematopoietic drug, wherein the drug comprises the pharmaceutical composition according to any one of claims 11 to 13.
